# EUROPEAN PATENT APPLICATION

(11) **EP 4 328 326 A1**
(43) Date of publication of application: **28.02.2024**
(21) Application number: 22192213.1
(22) Date of filing: 25.08.2022
(51) Int. Cl.: C12Q 1/6895, A01H 6/20, A01H 5/00, C12Q 1/00

(54) **GENE EXPRESSION MARKERS AS EARLY INDICATORS OF NITROGEN (N) DEFICIENCY**

(71) Applicant: Freie Universität Berlin, 14195 Berlin (DE)
(72) Inventor: KUNZE, Reinhard, 14532 Kleinmachnow (DE); SAFAVI-RIZI, Vajiheh, 04317 Leipzig (DE)
(74) Representative: Kilger, Ute

(57) **Abstract**

Subject matter of the present invention is a method for early determination of nitrogen (N) deficiency in rapeseed (*Brassica napus L*.) by using the gene expression of at least two marker genes of a set of marker genes, wherein said set comprises the *Brassica napus* expression marker genes BnaC03g77120D, BnaA02g27980D, BnaA03g40690D, BnaA06g37010D, BnaA06g22900D, BnaA03g40160D, BnaC07g37670D, BnaC05g14940D, BnaA09g04420D, BnaC01g35070D, BnaA02g32390D, BnaA07g29070D, BnaC01g14360D, BnaA09g28910D, BnaC07g41470D, BnaC06g30680D, BnaA02g02940D, BnaA03g48610D, BnaA01g24440D and BnaC01g03140D
wherein at least one of the two marker genes BnaA01g24440D (ELIP1) or BnaC01g03140D (DRL1) has to be used.

## Description

Subject matter of the present invention is a method for early indicating nitrogen (N) deficiency in rapeseed *(Brassica napus L.*) by using the gene expression of at least two marker genes of a whole set of *Brassica napus* marker genes, wherein at least one of the two genes BnaA01g24440D (ELIP1) or BnaC01g03140D (DRL1) has to be detected, and wherein the method comprises comparing the level of expression of said gene in a first biological sample taken from a plant with the level of expression of said gene in a second biological sample taken from a plant that was grown under optimal nitrogen supply or with a predetermined threshold,
and wherein a deviation of the level of expression of said gene in the first biological sample is indicative for said nitrogen-deficiency in rapeseed.

Rapeseed has low nitrogen use efficiency (NUE). Therefore, high N fertilizers, between 140 and 200 kg N ha-1 year-1 are applied to the field to achieve optimum yield (Avice et al. 2014). This has negative economic and environmental consequences. Traditional methods such as soil or plant tissue analysis are used to indicate fertilizer requirement. However, these indirect measurements are not precise enough. The aim of the studies for the present application was identification of gene expression markers as diagnostic tools to indicate N deficiency based on the physiological requirement of the plant for improved/optimized N fertilization under limited N condition, wherein the gene expression markers detect N deficiency earlier than the presently available methods.

Different rapeseed cultivars grown in various growth conditions (growth chamber, green house, hydroponic and field) were studied under adequate and limited N supply. In a step-by-step workflow using microarray and qPCR, 13 marker genes with robust expression level change under N deficiency were identified in all of the tested cultivars and growth conditions.

The results provide a proof-of-principle example of gene expression markers which can be used as physiological diagnostic tools for detection of N deficiency in rapeseed independent of cultivar and growth conditions.

This suggests a potential method for better management of fertilizer application not only in rapeseed but in other crops and stress conditions as well.

### Introduction

With a harvest of 76 million tons in 2017, rapeseed *(Brassica napus)* is worldwide the 3rd largest source of vegetable oil behind palm oil and soybean oil and the 2nd largest source of protein meal for animal feed. From 2003 to 2017 the worldwide production increased by 93%, in Europe by 124% and in North America by 181% (www.fao.org/faostat). The increase in rapeseed production is associated with the environmental burden of groundwater pollution with nitrate, because rapeseed has a low nitrogen use efficiency (NUE) (reviewed in Bouchet et al. 2016). During the vegetative stage, only little N is recovered from senescing early leaves because the N demand of reproductive sink organs is low. Consequently, dropped lower leaves have a high residual N content up to 2.5% of dry weight (Malagoli et al. 2005). Thus, of ~200 kg·ha-1 fertilizer N that is required for optimal seed yield, up to 100 kg·ha-1 N are released back to the soil by abscission of the lower leaves (Dejoux et al. 2000, Jackson 2000). Nitrogen remobilization is significantly more efficient in younger leaves in the upper canopy that senesce during pod development. At the time of abscission their nitrogen content is only 1% of dry weight (Malagoli et al. 2005).

To reduce the negative impacts of N fertilization on the environment it is critical to apply optimized quantities of N fertilizer. This is also economically important since both under- and overfertilization reduce yield production (Olfs et al. 2005). The improvement of N fertilizer application requires technical means for quick and accurate assessment of plant responses to variable nitrogen conditions (Yang et al. 2011). The nitrogen nutrition index (NNI) is an accurate measure of the nitrogen status in rapeseed (Colnenne et al. 2002). However, determining the NNI is laborious and destructive for the plant samples. A non-destructive and less laborious method is the estimation of relative leaf chlorophyll levels with a Soil Plant Analysis Development (SPAD) chlorophyll meter, as SPAD values correlate well with N content (Schulte aufm Erley et al. 2007).

Another approach to assess plant N status is remote sensing of canopy reflectance using ground-based sensors or satellite imaging.

Canopy sensors are more efficient for estimating the N status of crops on an entire field compared to SPAD chlorophyll meters, but the analysis of satellite images is time-consuming and may be too slow for N management if the image refreshment intervals are too long (reviewed by Munoz-Huerta et al. 2013). Aircraft- and drone-based screening is the most recent version of large scale monitoring of plants for N status assessment (Näsi et al. 2018). All these methods are useful when visible symptoms of N-deficiency (reduced chlorophyll) are detectable and are thus rather late diagnostic tools. In rapeseed remote sensing may have the additional limitation that the data reflect mostly the N content of young leaves in the top of the canopy, but N-deficiency symptoms appear first in the ground-level canopy leaves (Koeslin-Findeklee et al. 2015, Safavi-Rizi et al. 2018).

Precise N management requires detecting N-deficiency at an earlier stage before visible symptoms appear. Since molecular changes precede phenotypical changes, gene expression markers with N statusdependent expression levels can be useful tools to recognize N shortage at a very early stage, enabling to timely counteract upcoming N-deficiency symptoms. N-responsive expression markers have already been identified in maize and potato (Allender et al. 2010, Yang et al. 2011), but not yet in rapeseed or other crops.

Here, we report the identification of a set of robust *Brassica napus* expression marker genes that detect N deficiency at early stages in various rapeseed cultivars and growth conditions. In two independent N deficiency response transcriptome data sets commonly regulated genes in the double haploid rapeseed line Mozart, in the N-efficient cultivar Apex and the N-inefficient cultivar Capitol were identified (Koeslin-Findeklee et al. 2015, Safavi-Rizi et al. 2018). Among these genes 13 robust N deficiency markers genes were selected that show the qualitatively same transcriptional response to N deficiency also in four other rapeseed winter cultivars grown in a greenhouse and also in yet another field-grown winter cultivar.

It was the surprising finding of the present invention that these expression markers genes respond to N deficiency approximately 10 days prior to the appearance of leaf yellowing by chlorophyll degradation. The present invention describes the use of two or more of these expression markers genes for optimization of fertilizer application schemes.

### Detailed description of the invention

Subject matter according to the present invention is a method for early determination of nitrogen (N) deficiency in rapeseed *(Brassica napus L.*) by using the gene expression of at least two marker genes of a set of marker genes, wherein said set comprises the *Brassica napus* expression marker genes

| |
|---|
| BnaC03g77120D |
| BnaA02g27980D |
| BnaA03g40690D |
| BnaA06g37010D |
| BnaA06g22900D |
| BnaA03g40160D |
| BnaC07g37670D |
| BnaC05g14940D |
| BnaA09g04420D |
| BnaC01g35070D |
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |

wherein at least one of the two marker genes BnaA01g24440D (ELIP1) or BnaC01g03140D (DRL1) has to be used, and wherein the method comprises:
   - Determining the level of expression of said at least two marker genes in a biological sample taken from said rapeseed,
   - Comparing the level of expression of said at least two marker genes in a biological sample taken from said rapeseed with the level of expression of said genes in a second biological sample taken from a rapeseed plant that was grown under optimal nitrogen supply of preferably 120 kg N ha⁻¹ t to 150 kg N ha⁻¹ and/or with a predetermined threshold level of expression of said at least two marker genes,
wherein a deviation of the level of expression as determined by said comparison , which is an up regulation of BnaA09g04420D and/or BnaA02g32390D and/or BnaA07g29070D and/or BnaA02g02940D and/or BnaA03g48610D and/or a down regulation of BnaC03g77120D and/or BnaA02g27980D and/or BnaA03g40690D and/or BnaA06g37010D and/or BnaA06g22900D and/or BnaA03g40160D and/or BnaC07g37670D and/or BnaC05g14940D and/or BnaC01g35070D and/or BnaC01g14360D and/or BnaA09g28910D and/or BnaC07g41470D and/or BnaC06g30680D and/or BnaA01g24440D and/or BnaC01g03140D is indicative for a nitrogen deficiency in said rapeseed, and wherein the term early means before the leaves of the plant show phenotypical changes, in particular discoloration of the leaves due to nitrogen deficiency.

As used herein, the term "deviation" is defined as a change of gene expression when comparing the gene expression data of said first biological sample with the level of expression of said gene in said second biological sample, wherein deviation comprises an increase of expression and a decrease in expression.

The term "biological sample" refers to any sample that is believed to contain a substance of interest, in particular nucleic acid and optionally a protein or other biomolecule of interest. The term "sample" can encompass a solution (e.g. an aqueous solution), a cell, tissue, biopsy sample, powder, or a population of one or more of these biological materials. Samples may be taken from diverse parts of the plant such as the roots, shoots and leaves.

According to the present invention, the term "threshold" refers to a pre-determined gene expression level for each and every gene level of the afore mentioned marker genes in order to distinguish between rapeseed with or without nitrogen (N) deficiency.

In the context of the present application, the term optimal N supply is defined as a range of N supply of 120 kg N ha⁻¹ t to 150 kg N ha⁻¹ t, more preferred of 130 kg N ha⁻¹ t to 150 kg N ha⁻¹ t, even more preferred 150 kg N ha⁻¹ t according to conventional methods.

In the context of the present application, the term low N supply is defined as a range of 50 kg N ha⁻¹ t to 75 kg N ha⁻¹ t, more preferred 60 kg N ha⁻¹ t to 75 kg N ha⁻¹ t, most preferred 75 kg N ha⁻¹ t according to conventional methods.

The person skilled in the art would understand that N supply in a range between 75 kg N ha⁻¹ t to 120 kg N ha⁻¹ t according to conventional method is considered as moderate nitrogen deficiency and he would know how to determine the different stages of nitrogen deficiency.

As used herein, the term "early indicating" relates to the fact that the gene expression of said marker gene in the first biological sample responds to nitrogen deficiency before the leaves of the plant show phenotypical changes, in particular discoloration of the leaves due to nitrogen deficiency and/or before the rapeseed shows any other phenotypical signs of nitrogen deficiency.

Phenotypical signs of nitrogen deficiency comprise those plants, which are deficient in nitrogen have stunted growth, depending on the severity of the deficiency. Also, leaf growth is inhibited; younger leaves are inhibited in particular. Longitudinal shoot growth is inhibited, as well as the increase in thickness. Deficient plants often become pale green to yellowish-green due to inhibited chloroplast and chlorophyll synthesis. Leaves start to wither and dry out, turning yellowish brown to brown.

In the context of the present application, the term nitrogen deficiency is defined as a nitrogen level of < 90% of the optimal N level, or < 80 % of the optimal N level, or < 70% of the optimal N level, or < 60% of the optimal N level, or < 50% of the optimal N level as defined above.

As used herein, the term "gene expression" means the process by which a gene gets turned on in a cell to make RNA and proteins. Gene expression may be measured by determining the presence of the RNA, or the protein transcribed and translated from the RNA, or determining the function of the according protein in a cell. Gene expression determines the amount of mRNA synthesized from DNA. mRNA carries information for protein synthesis. However, gene expression does not necessarily correlate with the protein level as there are other regulatory processes involved between mRNA and protein synthesis such as splicing etc. In the context of the present invention, gene expression level preferably refers to the amount of mRNA synthesized from DNA.

In the context of the present application, the term marker gene is defined as a gene whose expression level changes based on a certain factor, in our case, nitrogen deficiency.

In the context of the present application, it is understood that a gene expression level can be obtained by any method and that the measurement level can be an absolute level, i.e., intensity level, a ratio, i.e., compared to a pre-determined threshold level of expression either of a reference gene or the gene itself, or a log ratio. For example, the pre-determined threshold level may comprise performing the same gene expression determination in a control sample of plants which grow under optimal N supply and comparing the same to the sample obtained from a plant growing under N deficiency.

The control sample may be from a single plant or a plurality of plants that are growing under optimal nitrogen supply.

In one embodiment the threshold may be pre-determined by comparing the level of expression of the marker gene in a first biological sample taken from rapeseed *(Brassica napus L.*) with the median of the level of expression of the marker gene in a biological sample obtained from an ensemble of predetermined samples in a randomly selected population of rapeseeds that were grown under optimal N conditions.

The terms "differentially expressed gene," "differential gene expression" and their synonyms, which are used interchangeably, refer to a gene whose expression is activated to a higher or lower level in a plant growing under N deficiency relative to its expression in a plant under normal or control conditions (optimal N supply).

It is also understood that a differentially expressed gene may be either activated or inhibited at the nucleic acid level or protein level or may be subject to alternative splicing to result in a different polypeptide product. Such differences may be evidenced by a change in mRNA levels, surface expression, secretion, or other partitioning of a polypeptide, for example. Differential gene expression may include a comparison of expression between two or more genes or their gene products, or a comparison of the ratios of the expression between two or more genes or their gene products, or even a comparison of two differently processed products of the same gene, which differ between plants growing under optimal nitrogen supply and plants growing under nitrogen deficiency. Differential expression includes both quantitative, as well as qualitative, differences in the temporal or cellular expression pattern in a gene or its expression products.

Differential gene expression can, for example, be a measure of the "fold difference" between two samples. Thus, for example, in one embodiment "differential gene expression" may be considered to be present when there is at least an about 1.2, 1.5. or 2-fold difference between the expression of a given gene of a rapeseed plant under normal and N deficient conditions. Differential gene expression can also be measured using a p-value. When using p-value in one embodiment, a marker gene is identified as being differentially expressed as between a first and second sample when the p-value is less than 0.1. In certain embodiments the p-value is less than 0. 05, while in others it may be lower.

As used herein, the phrase "fold difference" refers to a numerical representation of the magnitude difference between a measured value and a reference value for one or more of the marker genes of the invention. Fold difference is calculated mathematically by division of the numeric measured value with the numeric reference value.

As used herein, the term "up regulated," as used herein, refers to increased expression of a gene.

"Increased expression" refers to increasing (i.e., to a detectable extent) replication, transcription, and/or translation of any of the marker genes described herein since up-regulation of any of these processes results in concentration/amount increase of the polypeptide encoded by the gene (nucleic acid). Accordingly, "down regulation," or "decreased expression" as used herein, refers to decreased expression of a gene and/or its encoded polypeptide. The up regulation or down regulation of gene expression can be directly determined by detecting an increase or decrease, respectively, in the level of mRNA for the gene, or the level of protein expression of the gene-encoded polypeptide, using any suitable means known to the art, such as nucleic acid hybridization or antibody detection methods, respectively, and in comparison, to controls. In general, the variation in gene expression level is "statistically significant". Up or downregulation may be expressed as a fold-difference, e.g., genes or encoded proteins which demonstrate a e.g., 1.1-fold, or 1.2 fold, or 1.4 fold, or 1.6 fold, or 1.8 fold, or more increase or decrease in gene expression (as measured by RNA expression or protein expression), relative to a control or control level.

According to the invention, a "control level" or "control plant sample" or "reference level" means a separate baseline level measured in a comparable control plant, which is generally growing under optimal nitrogen supply.

A "reference value" can be an absolute value; a relative value; a value that has an upper and/or lower limit; a range of values; an average value; a median value, a mean value, or a value as compared to a particular control or baseline value.

One embodiment of the present application relates to a method for early indicating of nitrogen deficiency in rapeseed *(Brassica napus L.*) by using the gene expression of at least two marker genes of a set of marker genes, wherein said set comprises the *Brassica napus* expression marker genes

| |
|---|
| BnaA03g40160D |
| BnaC07g37670D |
| BnaC05g14940D |
| BnaA09g04420D |
| BnaC01g35070D |
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |

or said set comprises the *Brassica napus* expression marker genes

| |
|---|
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |

or said set comprises the *Brassica napus* expression marker genes

| |
|---|
| BnaC06g30680D |
| BnaA02g02940D |
| BnaA03g48610D |
| BnaA01g24440D |
| BnaC01g03140D |

or said set comprises the *Brassica napus* expression marker genes

| |
|---|
| BnaA01g24440D |
| BnaC01g03140D |

Another embodiment of the present application relates to a method for early determination of nitrogen deficiency in rapeseed *(Brassica napus L.*) by using the gene expression of at least two marker genes of a set of marker genes, wherein the gene expression of said marker genes respond to nitrogen deficiency before the leaves of the plant show phenotypical changes, in particular discoloration of the leaves due to nitrogen deficiency.

Nitrogen deficiency leads to a reduction of chlorophyll content of the plant which results in pale yellow color (chlorosis). Older leaves turn completely yellow. Nitrogen deficiency leads to a reduction of chlorophyll content of the plant (chlorosis) which results in yellowing of the leaves. Chlorosis starts from the older lower leaves and develops towards the upper part of the plant.

Subject matter of the present invention is a method for early determination of N deficiency in rapeseed *(Brassica napus L.*) by using the gene expression of at least two marker genes of a set of marker genes, wherein the expression profile is an RNA profile. Another embodiment of the present application relates to a method for early determination of nitrogen deficiency in rapeseed *(Brassica napus L.*) by using the gene expression of at least two marker genes of a set of marker genes, wherein the expression profile is an (m) RNA profile, and/or and RNA profile expressed in amount of (m)RNA.

In the context of the present application, the term RNA profile relates to gene expression profiling which is determined by measuring mRNA level of every single marker gene.

Another embodiment of the present application relates to a method for early determination of nitrogen deficiency in rapeseed *(Brassica napus L.*) by using the gene expression of at least two marker genes of a set of marker genes, wherein a deviation of the level of expression of said gene in the to be determined biological sample of a rapeseed is an upregulation or downregulation of gene expression compared to the gene expression of the marker gene in a second (reference) biological sample and/or with a predetermined threshold in a reference rapeseed plant.

Subject matter of the present application is also a method for early determination of nitrogen deficiency in rapeseed *(Brassica napus L.*) by using the gene expression of at least two marker genes of a set of marker genes, wherein an increase of the level of expression of said gene in the first biological sample compared to the level of expression of said gene in the second biological sample taken from a rapeseed plant that was grown under optimal nitrogen supply of preferably 120 kg N ha⁻¹ t to 150 kg N ha⁻¹ and/or with a predetermined threshold means the marker gene expression is up regulated, wherein a decrease of the gene expression level of said gene in the first biological sample compared to the level of expression of said gene in the second biological sample taken from a rapeseed plant that was grown under optimal nitrogen supply of preferably 120 kg N ha⁻¹ tto 150 kg N ha⁻¹ and/or with a predetermined threshold means the marker gene expression is downregulated,
and wherein an up regulation or down regulation of the gene expression of said marker gene is indicative for said nitrogen deficiency in rapeseed. First sample refers to the rapeseed to be determined and second sample refers to the reference plant.

Another embodiment of the present application relates to a method for monitoring nitrogen deficiency in rapeseed *(Brassica napus L.*) by using the gene expression of at least two marker genes of a set of marker genes, wherein said set comprises *Brassica napus* expression marker genes

| |
|---|
| BnaC03g77120D |
| BnaA02g27980D |
| BnaA03g40690D |
| BnaA06g37010D |
| BnaA06g22900D |
| BnaA03g40160D |
| BnaC07g37670D |
| BnaC05g14940D |
| BnaA09g04420D |
| BnaC01g35070D |
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |

wherein at least one of the two marker genes BnaA01g24440D (ELIP1) or BnaC01g03140D (DRL1) has to be used, and wherein the method comprises:
- Determining the level of expression of said at least two marker genes in a biological sample taken from said rapeseed,
- Comparing the level of expression of said at least two marker genes in a biological sample taken from said rapeseed with the level of expression of said genes in a second biological sample taken from a rapeseed plant that was grown under optimal nitrogen supply of preferably 120 kg N ha⁻¹ t to 150 kg N ha⁻¹ and/or with a predetermined threshold level of expression of said at least two marker genes,
wherein a deviation of the level of expression as determined by said comparison, which is an up regulation of BnaA09g04420D and/or BnaA02g32390D and/or BnaA07g29070D and/or BnaA02g02940D and/or BnaA03g48610D and/or a down regulation of BnaC03g77120D and/or BnaA02g27980D and/or BnaA03g40690D and/or BnaA06g37010D and/or BnaA06g22900D and/or BnaA03g40160D and/or BnaC07g37670D and/or BnaC05g14940D and/or BnaC01g35070D and/or BnaC01g14360D and/or BnaA09g28910D and/or BnaC07g41470D and/or BnaC06g30680D and/or BnaA01g24440D and/or BnaC01g03140D is indicative for a nitrogen deficiency in said rapeseed,
and wherein the term early means before the leaves of the plant show phenotypical changes, in particular discoloration of the leaves due to nitrogen deficiency.

Another embodiment of the present application relates to a method for determining the need of nitrogen fertilization in rapeseed *(Brassica napus L.*) by using the gene expression of at least two marker genes of a set of marker genes, wherein gene expression of said genes is nitrogen responsive and is used as an early indicator for the nitrogen status in the plant, wherein said set comprises *Brassica napus* expression marker genes:

| |
|---|
| BnaC03g77120D |
| BnaA02g27980D |
| BnaA03g40690D |
| BnaA06g37010D |
| BnaA06g22900D |
| BnaA03g40160D |
| BnaC07g37670D |
| BnaC05g14940D |
| BnaA09g04420D |
| BnaC01g35070D |
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |

wherein at least one of the two marker genes BnaA01g24440D (ELIP1) or BnaC01g03140D (DRL1) has to be used, and wherein the method comprises:
a) Determining the level of expression of said at least two marker genes in a biological sample taken from said rapeseed,
b) Comparing the level of expression of said at least two marker genes in a biological sample taken from said rapeseed with the level of expression of said genes in a second biological sample taken from a rapeseed plant that was grown under optimal nitrogen supply of preferably 120 kg N ha⁻¹ t to 150 kg N ha⁻¹ and/or with a predetermined threshold level of expression of said at least two marker genes,
   wherein a deviation of the level of expression as determined by said comparison, which is an up-regulation of BnaA09g04420D and/or BnaA02g32390D and/or BnaA07g29070D and/or BnaA02g02940D and/or BnaA03g48610D and/or a downregulation of BnaC03g77120D and/or BnaA02g27980D and/or BnaA03g40690D and/or BnaA06g37010D and/or BnaA06g22900D and/or BnaA03g40160D and/or BnaC07g37670D and/or BnaC05g14940D and/or BnaC01g35070D and/or BnaC01g14360D and/or BnaA09g28910D and/or BnaC07g41470D and/or BnaC06g30680D and/or BnaA01g24440D and/or BnaC01g03140D is indicative for a nitrogen deficiency in said rapeseed,
   and wherein the term early means before the leaves of the plant show phenotypical changes, in particular discoloration of the leaves due to nitrogen deficiency,
c) Selecting a plant that displays nitrogen deficiency according to step b)
d) Optimizing the application with nitrogen for the selected plant by increasing the nitrogen amount.

Another embodiment of the present application relates to a method for marker genes guided plant fertilization in rapeseed *(Brassica napus L.*) by using the gene expression of at least two marker genes of a set of gene expression marker genes according to the present invention, wherein gene expression of said genes is nitrogen responsive and is used as an early indicator for the nitrogen status in the plant, wherein said set comprises *Brassica napus* expression marker genes:

| |
|---|
| BnaC03g77120D |
| BnaA02g27980D |
| BnaA03g40690D |
| BnaA06g37010D |
| BnaA06g22900D |
| BnaA03g40160D |
| BnaC07g37670D |
| BnaC05g14940D |
| BnaA09g04420D |
| BnaC01g35070D |
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |

wherein at least one of the gene expression marker genes BnaA01g24440D (ELIP1) or BnaC01g03140D (DRL1) from *Brassica napus L.* has to be used, and wherein the method comprises
a) Determining the level of expression of said at least two marker genes in a biological sample taken from said rapeseed,
b) Comparing the level of expression of said at least two marker genes in a biological sample taken from said rapeseed with the level of expression of said genes in a second biological sample taken from a rapeseed plant that was grown under optimal N supply of preferably 120 kg N ha⁻¹ t to 150 kg N ha⁻¹ and/or with a predetermined threshold level of expression of said at least two marker genes,
   wherein a deviation of the level of expression as determined by said comparison, which is an up regulation of BnaA09g04420D and/or BnaA02g32390D and/or BnaA07g29070D and/or BnaA02g02940D and/or BnaA03g48610D and/or a down-regulation of BnaC03g77120D and/or BnaA02g27980D and/or BnaA03g40690D and/or BnaA06g37010D and/or BnaA06g22900D and/or BnaA03g40160D and/or BnaC07g37670D and/or BnaC05g14940D and/or BnaC01g35070D and/or BnaC01g14360D and/or BnaA09g28910D and/or BnaC07g41470D and/or BnaC06g30680D and/or BnaA01g24440D and/or BnaC01g03140D is indicative for a nitrogen deficiency in said rapeseed,
   and wherein the term early means before the leaves of the plant show phenotypical changes, in particular discoloration of the leaves turn yellow due to nitrogen deficiency
c) Selecting a plant that displays nitrogen deficiency according to step b).
d) Optimizing the application with nitrogen for the selected plant by increasing the N amount.

Subject matter of the present invention is a method for monitoring nitrogen deficiency in rapeseed (*Brassica napus L.*) by performing a method for early determination of nitrogen (N) deficiency in rapeseed at least twice and wherein said method is used to monitor the response of a plant being treated for nitrogen deficiency by supplying nitrogen.

An embodiment of the present invention is a method for monitoring nitrogen deficiency in rapeseed *(Brassica napus L.*) by performing a method for early determination of nitrogen (N) deficiency in rapeseed at least twice, preferably at least three times, preferably at least four times, more preferably at least five times, more preferably at least six times, more preferably seven times, more preferably eight times, more preferably nine times and most preferably at least ten times.

Another embodiment of the present invention is a method for monitoring N deficiency in rapeseed (*Brassica napus L.*) by performing a method for early determination of nitrogen (N) deficiency in rapeseed five times. Typically, the nitrogen content in leaves is determined. A common method to determine the nitrogen level in plants is tissue analysis by e.g. the Kjedahl digestion or the Dumas combustion. Alternatively, noninvasive methods can be used to determine the nitrogen content such as different leaf chlorophyll meters, e.g. SPAD-502, and chlorophyll fluorescence meters. Alternatively, the nitrogen content can be determined using a UAV (unmanned Aerial Vehicle), biosensors or by measuring the nitrate Sap content and electrical variables such as reflectometers and nitrate test strips, ion selective electrodes and impedance measurements. However, these methods do not allow an early determination of nitrogen deficiency. In an embodiment of the present invention, the plants are monitored 2 weeks prior to the application of nitrogen by sampling different tissues. At this time point, a method according to the present invention is performed. In addition, the nitrogen content in the plant may be measured according to standard methods known in the art. Thereby, the method according to the present invention enables the person skilled in the art to monitor a nitrogen deficiency as early as possible.

Monitoring the response of a plant being treated for nitrogen deficiency by supplying nitrogen according to the present invention comprises observing phenotypical changes of the coloration of the plant. Upon the application of nitrogen, the plant will absorb the nutrient as soon as it is available and the leaves will start to change from pale yellow to healthy green. However, the person skilled in the art would be aware that other factors such as the pH rate and nutrient content of the soil have to be taken into account. Basic sources of Nitrogen supply comprise compost, animal manure, horn, bone, fish or blood meal, nettle slag, green mature, tree leaves, ashes and others. Alternatively chemical fertilizers such as NPK, nitrolime, ammonium nitrate and urea can be used. In an embodiment of the present invention, monitoring the response of a plant being treated for nitrogen deficiency by supplying nitrogen comprises monitoring the need of the plant for nitrogen supply. By performing the method according to the present invention, the expression of the marker genes can be correlated with the amount of nitrogen and enables the person skilled in the art to determine the plant's need for nitrogen supply, but eventually also to determine the best time point to supply nitrogen. Here, different growth phases of the plant can be taken into account as they also influence the plant's need for nitrogen. In some embodiments of the present invention, monitoring the response of a plant being treated for nitrogen deficiency by supplying nitrogen comprises applying fewer amounts of nitrogen to the plant depending on the plant's need for nitrogen supply.

Subject matter of the present invention is a method for determining the need of plant nitrogen fertilization in rapeseed *(Brassica napus L.*) by performing a method for early determination of nitrogen (N) deficiency in rapeseed, and thereby selecting a rapeseed that displays nitrogen deficiency and preferably optimizing the application or fertilization with nitrogen for the selected plant by increasing or reducing the nitrogen amount.

Increasing or decreasing the N amount refers to increasing the amount of nitrogen available as NO₃ and amounts of nitrogen available as NH₄. In this context, increasing the nitrogen amount refers to applying a greater amount of nitrogen to said plant. The person skilled in the art would know how to calculate the correct dosage to the plants in order to avoid a nitrogen toxicity or to aggravate the nitrogen deficiency. In general, the person skilled in the art would know how to determine the critical nitrogen application rate, which takes into account the yield and an acceptable amount of nitrate leaching.

Subject-matter of the present application is also an assay for early determination of nitrogen (N) deficiency in rapeseed (*Brassica napus L.*) comprising *Brassica napus* expression marker genes:

| |
|---|
| BnaC03g77120D |
| BnaA02g27980D |
| BnaA03g40690D |
| BnaA06g37010D |
| BnaA06g22900D |
| BnaA03g40160D |
| BnaC07g37670D |
| BnaC05g14940D |
| BnaA09g04420D |
| BnaC01g35070D |
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |

, wherein the assay comprises preferably an assay for RNA extraction and a microarray or a PCR-based method.

A "PCR-based method" refers to methods comprising a polymerase chain reaction (PCR). A PCR is a method of exponentially amplifying nucleic acids, such as DNA or RNA by enzymatic replication in vitro by using at least one primer pair of forward and reverse primers. For RNA amplification, a reverse transcription is typically used as a first step. PCR-based methods also comprise kinetic or quantitative PCR (qPCR) which is well suited for the analysis of expression levels. When determining the expression levels of marker genes, a PCR based method may for example be used to detect the presence of a given RNA molecule by either reverse transcription of the transcriptome into cDNA with help of a reverse transcriptase enzyme, or by detecting the presence of a given cDNA with help of respective primers. This approach is commonly known as reverse transcriptase PCR (RT PCR). The term "PCR based method" comprises both end-point PCR applications as well as kinetic/real time PCR techniques applying special fluorophors or intercalating dyes which emit fluorescent signals as a function of amplified target and allow monitoring and quantification of the target. Quantification methods could be either absolute by external standard curves or relative to a comparative internal standard.

In particular, the following devices and components may be used: Real-Time PCR device or any other device suitable for performing a PCR-based method including marker gene specific primers, fluorescent dyes such as SYBR Green ^{®} and the like. The amplification is typically carried out using amplification primers, a suitable buffer system, nucleotides, and DNA polymerase enzyme such as thermostable DNA polymerase, e.g. the Taq-Polymerase. The person skilled in the art can then easily determine, for any given sample, primer sequence, and reaction condition, how many cycles are sufficient to determine the presence of a given target nucleic acid.

In a particular embodiment of the present invention, combined thermal cycling and fluorescence detecting devices can be used for precise quantification of target nucleic acids. In some embodiments, fluorescent signals can be detected and displayed during and/or after one or more thermal cycles, thus permitting monitoring of amplification products as the reactions occur in "real-time." In certain embodiments, one can use the amount of amplification product and number of amplification cycles to calculate how much of the target nucleic acid sequence was in the sample prior to amplification. Typically, the generated PCR-based method data are analysed via the double delta Ct analysis and the relative standard curve method (Pfaffl method). These methods are very well known in the art.

In an embodiment of the present invention the PCR based method or the Microarray is automated.

The term "microarray," as used herein, refers to an ordered array of spots presented for binding to ligands of interest. A microarray consists of at least two spots. The ligands of interest include, but are not limited to, nucleic acids (e.g., molecular beacons, aptamers, locked nucleic acids, peptide nucleic acids), proteins, peptides, polysaccharides, antibodies, antigens, viruses, and bacteria. In an embodiment of the present invention the microarray is a DNA microarray. The method is very well known in the art, see e.g. Stears et al., Nat Med, 2003, doi: 10.1038/nm0103-140.

In another embodiment, analysing the data generated from experiments on microarrays comprises applying an algorithm. Interpreting data generated from experiments on microarrays is performed by using microarray analysis techniques. Such experiments can generate very large amounts of data and may therefore be analysed with the help of mathematical algorithms. The person skilled in the art would be aware which statistical analysis can be applied. These include e.g. methods for aggregation and normalization, the identification of significant differential expression, the significance analysis of microarrays (SAM) and a Clustering analysis.

However, any suitable method for determining the marker genes according to the present invention can be used and comprises but is not limited to different RNA sequencing methods, loop mediated isothermal amplification (LAMP) and sequencing technologies such as NGS.

Subject-matter of the present application is also a use of gene expression of at least two gene expression marker genes of a set of gene expression marker genes for monitoring early nitrogen (N) deficiency in rapeseed *(Brassica napus L.),*
wherein the gene expression of said gene expression marker genes is nitrogen responsive and is used as an early indicator for the nitrogen status in the plant, wherein said set comprises *Brassica napus* expression marker genes

| |
|---|
| BnaC03g77120D |
| BnaA02g27980D |
| BnaA03g40690D |
| BnaA06g37010D |
| BnaA06g22900D |
| BnaA03g40160D |
| BnaC07g37670D |
| BnaC05g14940D |
| BnaA09g04420D |
| BnaC01g35070D |
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |

wherein at least one of the gene expression marker genes BnaA01g24440D (ELIP1) or BnaC01g03140D (DRL1) from *Brassica napus L.* has to be detected.

It is also in scope of the present invention that the claimed method for early determination of nitrogen deficiency according to the present invention can also be transferred to other crops. The person skilled in the art would be aware that respective homologue genes to the ones disclosed in the present invention may exist and that these homologue genes could be used to apply the disclosed methods accordingly.

### References

Allender, C. J. and G. J. King (2010). "Origins of the amphiploid species Brassica napus L. investigated by chloroplast and nuclear molecular markers." BMC Plant Biology 10: 54.

Arvidsson, S., M. Kwasniewski, D. M. Riano-Pachon and B. Mueller-Roeber (2008). "QuantPrime--a flexible tool for reliable high-throughput primer design for quantitative PCR." BMC Bioinformatics 9: 465.

Avice, J. C. and P. Etienne (2014). "Leaf senescence and nitrogen remobilization efficiency in oilseed rape (Brassica napus L.)." J Exp Bot 65(14): 3813-3824.

Bouchet, A. S., A. Laperche, C. Bissuel-Belaygue, C. Baron, J. Morice, M. Rousseau-Gueutin, J. E. Dheu, P. George, X. Pinochet, T. Foubert, O. Maes, D. Dugue, F. Guinot and N. Nesi (2016). "Genetic basis of nitrogen use efficiency and yield stability across environments in winter rapeseed." Bmc Genetics 17**.**

Buchanan-Wollaston, V., T. Page, E. Harrison, E. Breeze, P. O. Lim, H. G. Nam, J. F. Lin, S. H. Wu, J. Swidzinski, K. Ishizaki and C. J. Leaver (2005). "Comparative transcriptome analysis reveals significant differences in gene expression and signalling pathways between developmental and dark/starvation-induced senescence in Arabidopsis." Plant J 42(4): 567-585.

Casazza, A. P., S. Rossini, M. G. Rosso and C. Soave (2005). "Mutational and expression analysis of ELIP1 and ELIP2 in Arabidopsis thaliana." Plant Molecular Biology 58(1): 41-51.

Chandler, M., F. de la Cruz, F. Dyda, A. B. Hickman, G. Moncalian and B. Ton-Hoang (2013). "Breaking and joining single-stranded DNA: the HUH endonuclease superfamily." Nat Rev Microbiol 11(8): 525-538.

Chen, C., A. D. Farmer, R. J. Langley, J. Mudge, J. A. Crow, G. D. May, J. Huntley, A. G. Smith and E. F. Retzel (2010). "Meiosis-specific gene discovery in plants: RNA-Seq applied to isolated Arabidopsis male meiocytes." BMC Plant Biology 10: 280.

Colnenne, C., J. M. Meynard, R. Roche and R. Reau (2002). "Effects of nitrogen deficiencies on autumnal growth of oilseed rape." European Journal of Agronomy 17(1): 11-28.

Craw, P. and W. Balachandran (2012). "Isothermal nucleic acid amplification technologies forpoint-ofcare diagnostics: a critical review." Lab Chip 12(14): 2469-2486.

Czechowski, T., R. P. Bari, M. Stitt, W. R. Scheible and M. K. Udvardi (2004). "Real-time RT-PCR profiling of over 1400 Arabidopsis transcription factors: unprecedented sensitivity reveals novel rootand shoot-specific genes." Plant Journal 38(2): 366-379.

Dejoux, J.-F., S. Recous, J. M. Meynard, I. Trinsoutrot and P. Leterme (2000). "The fate of nitrogen from winter-frozen rapeseed leaves: mineralization, fluxes to the environment and uptake by rapeseed crop in spring." Plant and Soil 218(1-2): 257-272.

Diers, B. W. and T. C. Osborn (1994). "Genetic diversity of oilseedBrassica napus germ plasm based on restriction fragment length polymorphisms." Theor Appl Genet 88(6-7): 662-668.

Franzaring, J., S. Weller, I. Schmid and A. Fangmeier (2011). "Growth, senescence and water use efficiency of spring oilseed rape (Brassica napus L. cv. Mozart) grown in a factorial combination of nitrogen supply and elevated CO2." Environmental and Experimental Botany 72(2): 284-296.

Gill, P. and A. Ghaemi (2008). "Nucleic acid isothermal amplification technologies: a review." Nucleosides Nucleotides Nucleic Acids 27(3): 224-243.

Guo, Y., Z. Cai and S. Gan (2004). "Transcriptome of Arabidopsis leaf senescence." Plant Cell and Environment 57: 521-549.

Jackson, G. D. (2000). "Effects of nitrogen and sulfur on canola yield and nutrient uptake." Agronomy Journal 92(4): 644-649.

Koeslin-Findeklee, F., M. A. Becker, E. van der Graaff, T. Roitsch and W. J. Horst (2015). "Differences between winter oilseed rape (Brassica napus L.) cultivars in nitrogen starvation-induced leaf senescence are governed by leaf-inherent rather than root-derived signals." J Exp Bot 66(13): 3669-3681. Koeslin-Findeklee, F., V. S. Rizi, M. A. Becker, S. Parra-Londono, M. Arif, S. Balazadeh, B. Mueller-Roeber, R. Kunze and W. J. Horst (2015). "Transcriptomic analysis of nitrogen starvation- and cultivarspecific leaf senescence in winter oilseed rape (Brassica napus L.)." Plant Sci 233: 174-185.

Liu, J., J. Li, H. Wang, Z. Fu, J. Liu and Y. Yu (2011). "Identification and expression analysis of ERF transcription factor genes in petunia during flower senescence and in response to hormone treatments." J Exp Bot 62(2): 825-840.

Malagoli, P., P. Laine, L. Rossato and A. Ourry (2005). "Dynamics of nitrogen uptake and mobilization in field-grown winter oilseed rape (Brassica napus) from stem extension to harvest. II. An 15N-labellingbased simulation model of N partitioning between vegetative and reproductive tissues." Ann Bot 95(7): 1187-1198.

Matsui, K., Y. Umemura and M. Ohme-Takagi (2008). "AtMYBL2, a protein with a single MYB domain, acts as a negative regulator of anthocyanin biosynthesis in Arabidopsis." Plant J 55(6): 954-967.

Munoz-Huerta, R. F., R. G. Guevara-Gonzalez, L. M. Contreras-Medina, I. Torres-Pacheco, J. Prado-Olivarez and R. V. Ocampo-Velazquez (2013). "A review of methods for sensing the nitrogen status in plants: advantages, disadvantages and recent advances." Sensors (Basel) 13(8): 10823-10843.

Näsi, R., N. Viljanen, J. Kaivosoja, K. Alhonoja, T. Hakala, L. Markelin and E. Honkavaara (2018). "Estimating Biomass and Nitrogen Amount of Barley and Grass Using UAV and Aircraft Based Spectral and Photogrammetric 3D Features." Remote Sensing 10(7).

Olfs, H.-W., K. Blankenau, F. Brentrup, J. Jasper, A. Link and J. Lammel (2005). "Soil- and plant-based nitrogen-fertilizer recommendations in arable farming." Journal of Plant Nutrition and Soil Science 168(4): 414-431.

Ren, G., K. An, Y. Liao, X. Zhou, Y. Cao, H. Zhao, X. Ge and B. Kuai (2007). "Identification of a novel chloroplast protein AtNYE1 regulating chlorophyll degradation during leaf senescence in Arabidopsis." Plant Physiology 144(3): 1429-1441.

Safavi-Rizi, V., J. Franzaring, A. Fangmeier and R. Kunze (2018). "Divergent N Deficiency-Dependent Senescence and Transcriptome Response in Developmentally Old and Young Brassica napus Leaves." Front Plant Sci 9: 48.

Scheible, W. R., R. Morcuende, T. Czechowski, C. Fritz, D. Osuna, N. Palacios-Rojas, D. Schindelasch, O. Thimm, M. K. Udvardi and M. Stitt (2004). "Genome-wide reprogramming of primary and secondary metabolism, protein synthesis, cellular growth processes, and the regulatory infrastructure of Arabidopsis in response to nitrogen." Plant Physiol 136(1): 2483-2499.

Schulte aufm Erley, G., K.-A. Wijaya, A. Ulas, H. Becker, F. Wiesler and W. J. Horst (2007). "Leaf senescence and N uptake parameters as selection traits for nitrogen efficiency of oilseed rape cultivars." Physiologia Plantarum 130(4): 519-531.

Shirzadegan, M., P. Christie and J. R. Seemann (1991). "An efficient method for isolation of RNA from tissue cultured plant cells." Nucleic Acids Res 19(21): 6055.

Song, Y., C. Yang, S. Gao, W. Zhang, L. Li and B. Kuai (2014). "Age-Triggered and Dark-Induced Leaf Senescence Require the bHLH Transcription Factors PIF3, 4, and 5." Molecular Plant 7(12): 1776-1787.

Uauy, C., A. Distelfeld, T. Fahima, A. Blechl and J. Dubcovsky (2006). "A NAC Gene regulating senescence improves grain protein, zinc, and iron content in wheat." Science 314(5803): 1298-1301. Winter, D., B. Vinegar, H. Nahal, R. Ammar, G. V. Wilson and N. J. Provart (2007). "An "Electronic Fluorescent Pictograph" browser for exploring and analyzing large-scale biological data sets." PLoS One 2(1): e718.

Yang, X. S., J. Wu, T. E. Ziegler, X. Yang, A. Zayed, M. S. Rajani, D. Zhou, A. S. Basra, D. P. Schachtman, M. Peng, C. L. Armstrong, R. A. Caldo, J. A. Morrell, M. Lacy and J. M. Staub (2011). "Gene expression biomarkers provide sensitive indicators of in planta nitrogen status in maize." Plant Physiology 157(4): 1841-1852.

Ye, H., L. Li, H. Guo and Y. Yin (2012). "MYBL2 is a substrate of GSK3-like kinase BIN2 and acts as a corepressor of BES1 in brassinosteroid signaling pathway in Arabidopsis." Proc Natl Acad Sci U S A 109(49): 20142-20147.

Zhang, C., M. Y. Bai and K. Chong (2014). "Brassinosteroid-mediated regulation of agronomic traits in rice." Plant Cell Rep 33(5): 683-696.

The following embodiments are subject of the present invention:
1. A method for early determination of nitrogen (N) deficiency in rapeseed *(Brassica napus L.*) by using the gene expression of at least two marker genes of a set of marker genes, wherein said set comprises the *Brassica napus* expression marker genes

| |
|---|
| BnaC03g77120D |
| BnaA02g27980D |
| BnaA03g40690D |
| BnaA06g37010D |
| BnaA06g22900D |
| BnaA03g40160D |
| BnaC07g37670D |
| BnaC05g14940D |
| BnaA09g04420D |
| BnaC01g35070D |
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |

wherein at least one of the two marker genes BnaA01g24440D (ELIP1) or BnaC01g03140D (DRL1) has to be used, and wherein the method comprises:
- Determining the level of expression of said at least two marker genes in a biological sample taken from said rapeseed,
- Comparing the level of expression of said at least two marker genes in a biological sample taken from said rapeseed with the level of expression of said genes in a second biological sample taken from a rapeseed plant that was grown under optimal nitrogen supply of preferably 120 kg N ha⁻¹ t to 150 kg N ha⁻¹ and/or with a predetermined threshold level of expression of said at least two marker genes,

wherein a deviation of the level of expression as determined by said comparison, which is an up regulation of BnaA09g04420D and/or BnaA02g32390D and/or BnaA07g29070D and/or BnaA02g02940D and/or BnaA03g48610D and/or a down regulation of BnaC03g77120D and/or BnaA02g27980D and/or BnaA03g40690D and/or BnaA06g37010D and/or BnaA06g22900D and/or BnaA03g40160D and/or BnaC07g37670D and/or BnaC05g14940D and/or BnaC01g35070D and/or BnaC01g14360D and/or BnaA09g28910D and/or BnaC07g41470D and/or BnaC06g30680D and/or BnaA01g24440D and/or BnaC01g03140D, is indicative for a nitrogen deficiency in said rapeseed,
and wherein the term early means before the leaves of the plant show phenotypical changes, in particular discoloration of the leaves due to nitrogen deficiency.
2. The method for early determination of nitrogen deficiency in rapeseed *(Brassica napus L.*) by using the gene expression of at least two marker genes of a set of marker genes according to embodiment 1, wherein the expression profile is an RNA profile.
3. The method for early determination of nitrogen deficiency in rapeseed (*Brassica napus L.*) by using the gene expression of at least two marker genes of a set of marker genes according to embodiments 1 to 2, wherein an increase of the level of expression of said gene in the biological sample compared to the level of expression of said gene in the second biological sample taken from a rapeseed plant that was grown under optimal nitrogen supply of preferably 120 kg N ha⁻¹ t to 150 kg N ha⁻¹ and/or with a predetermined threshold means the marker gene expression is up-regulated,
wherein a decrease of the gene expression level of said gene in the biological sample compared to the level of expression of said gene in the second biological sample taken from a rapeseed plant that was grown under optimal nitrogen supply of preferably 120 kg N ha⁻¹ t to 150 kg N ha⁻¹ and/or with a predetermined threshold means the marker gene expression is downregulated,
and wherein an up regulation or down regulation of the gene expression of said gene is indicative for said nitrogen deficiency in rapeseed.
4. A method for monitoring N deficiency in rapeseed *(Brassica napus L.*) by performing a method for early determination of nitrogen (N) deficiency in rapeseed according to embodiments 1-3 at least twice and wherein said method is used to monitor the response of a plant being treated for nitrogen deficiency by supplying nitrogen.
5. A method for determining the need of plant N fertilization in rapeseed (*Brassica napus L.*) by performing a method for early determination of nitrogen (N) deficiency in rapeseed according to any of embodiments 1-4, and thereby selecting a rapeseed that displays nitrogen deficiency and preferably optimizing the application with nitrogen for the selected plant by increasing or reducing the nitrogen amount.
6. An assay for early determination of nitrogen (N) deficiency in rapeseed (*Brassica napus L.*) comprising *Brassica napus* expression marker genes:

| |
|---|
| BnaC03g77120D |
| BnaA02g27980D |
| BnaA03g40690D |
| BnaA06g37010D |
| BnaA06g22900D |
| BnaA03g40160D |
| BnaC07g37670D |
| BnaC05g14940D |
| BnaA09g04420D |
| BnaC01g35070D |
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |

, wherein the assay preferably also comprises an assay for RNA extraction and a microarray or a PCR based method.
7. Use of gene expression of at least two marker genes of a set of marker genes for detecting and/or monitoring early nitrogen (N) deficiency in rapeseed *(Brassica napus L.),*
wherein the gene expression of said marker genes is nitrogen responsive and is used as an early indicator for the nitrogen status in the plant, wherein said set comprises *Brassica napus* expression marker genes

| |
|---|
| BnaC03g77120D |
| BnaA02g27980D |
| BnaA03g40690D |
| BnaA06g37010D |
| BnaA06g22900D |
| BnaA03g40160D |
| BnaC07g37670D |
| BnaC05g14940D |
| BnaA09g04420D |
| BnaC01g35070D |
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |

wherein at least one of the marker genes BnaA01g24440D (ELIP1) or BnaC01g03140D (DRL1) from *Brassica napus L.* has to be detected.

### Figure description

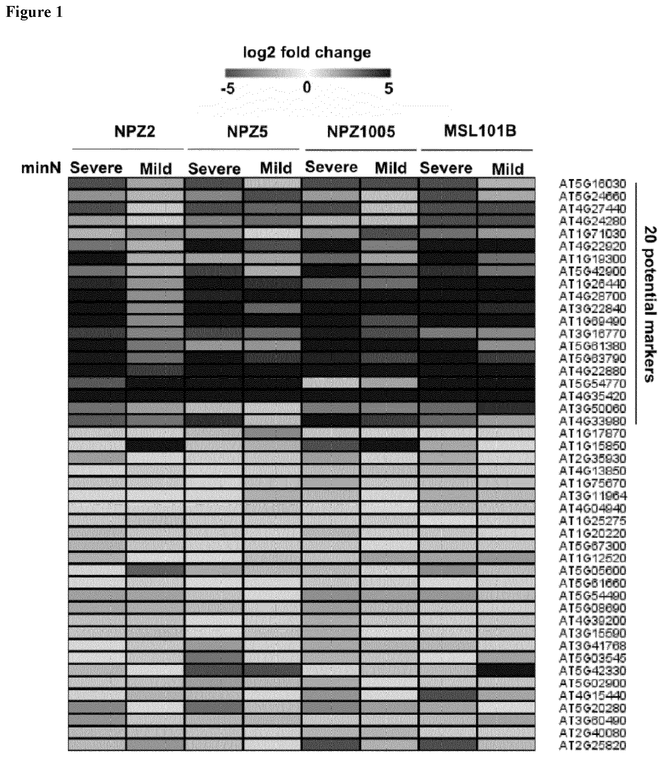

**Figure 1****:** Heatmap of gene expression fold changes in response to minN (severe and mild) in 4 different cultivars (NPZ2, NPZ5, NPZ1005 and MSL101B) grown in greenhouse. 20 out of 46 genes, showed similar expression changes in all cultivars under both sever and mild minN conditions (marked as 20 potential markers). Fold changes are calculated as the expression level of each gene under minN (Sever: 250 and mild: 500 mg N) relative to optN (1000 mg N). The oldest lowest leaf at 27 DAT was used for the RT-PCR analysis. Relative transcript levels were normalized to UP1 and UBC9 as reference genes. Down and upregulated markers are shown by light and darf grey colors, respectively. Heatmap is generated using MultiExperiment Viewer (MeV) (www.tm4.org/mev). DAT: days after transplanting.

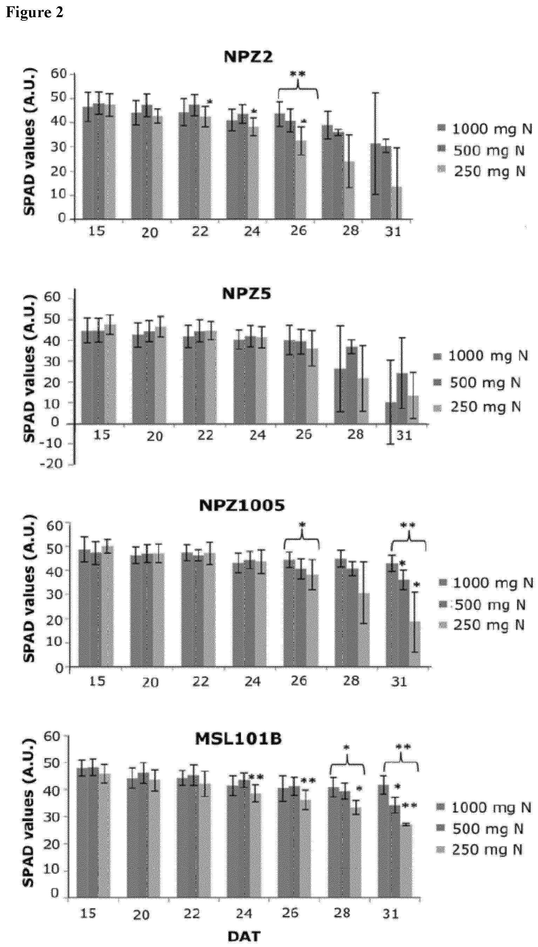

**Figure 2****:** SPAD values of the oldest leaf belonging to different rapeseed winter cultivars grown in green house. Cultivars NPZ2, NPZ5, NPZ1005 and MSL101B were treated with three N concentrations (optN, 1000mg N and minN: mild, 500 mg N and severe, 50 mg N). Asterisks indicate statistically significant differences as determined by Student's *t*-test (^{∗}P < 0.05 and ^{∗∗}P < 0.01). Each value is the mean of the 4 biological replicates (n=4) and the error bars correspond to the SD. DAT: days after transplanting. Validation of potential marker genes in the field grown samples

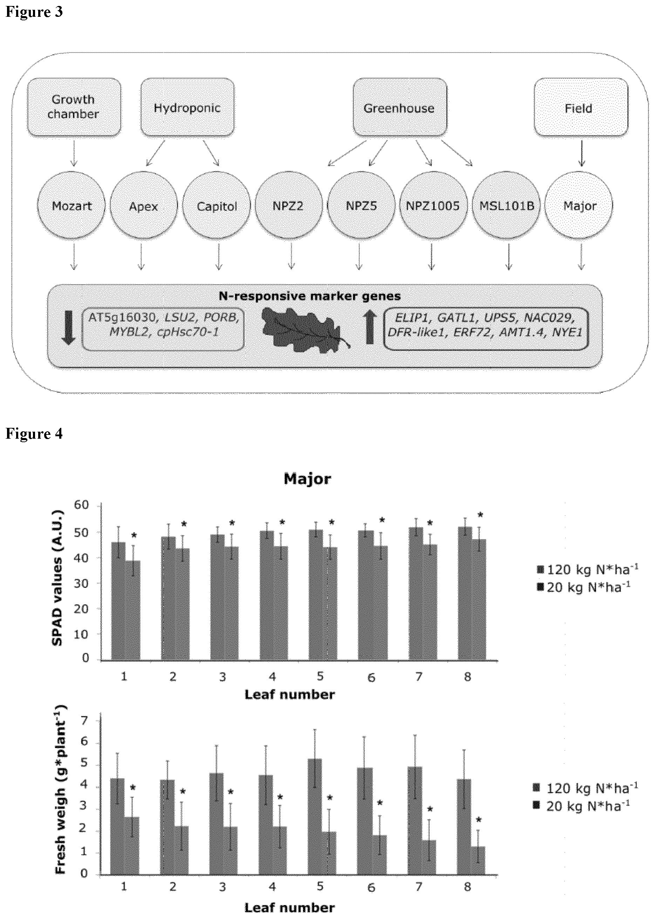

**Figure 3****:** A schematic representation of cultivars, growth conditions and final marker genes identified in this study. Rectangular boxes represent the growth conditions and circles show the cultivars which was grown under that condition. Up and downregulated N-responsive genes are indicated by an arrow, respectively.
**Figure 4****:** SPAD values (A), fresh weight (B) of rapeseed, major cultivar, grown in the field under normal (120 N ha⁻¹) and low (20 kg N ha⁻¹). Asterisks indicate statistically significant differences as determined by Student's *t*-test (^{∗}P < 0.05 and ^{∗∗}P < 0.01). Each value is the mean of the three biological replicates (n=3) and the error bars correspond to the STD.

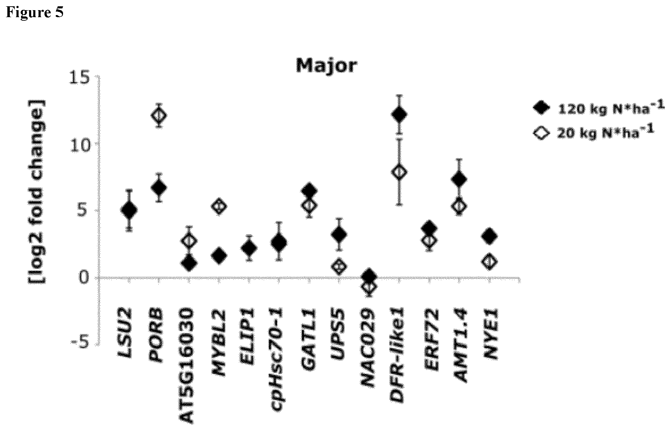

**Figure 5****:** Relative expression of 13 marker genes with distinct expression levels under different N conditions in the field grown cultivar Major. Plants were grown under optN (120 kg N/ha) and minN (120 kg N/ha). The 4th lowest leaf at early flowering stage was used for the RT-PCR analysis. Relative transcript levels were normalized to UP1 and UBC9. Error bars represent SD (n=4).

### Examples

### Example 1

### Identification of candidate N-deficiency marker genes

In a prior microarray study, in leaves of growth chamber-grown spring cultivar 'Mozart' hundreds of genes were identified that were differentially regulated under mild N-deficiency (NL: 75 kg N·ha⁻¹) compared to plants grown under optimal N supply (NO: 150 kg N·ha⁻¹) (Safavi-Rizi et al. 2018). To identify robust expression markers for N-deficiency in 'Mozart' we selected from these data genes which were up- or downregulated >10-fold in NL-plants and <3-fold in NO-plants (or vice versa) in leaf #4 during one of the three developmental intervals 78-85 days after sowing (DAS), 85-92 DAS or 92-99 DAS. A total of 130 genes fulfilled these criteria. Among these, 35 genes showed overall different expression profiles in NL versus NO leaves during development in all four time points. In another study, 12 N-sensitive and cultivar specific genes were identified in hydroponically grown winter cultivars Apex (N-efficient) and Capitol (N-inefficient) (Koeslin-Findeklee etal. 2015). As one gene was shared in the two groups, the two gene sets comprised in total 46 potential marker genes.

### Validation of candidate N-deficiency marker genes in greenhouse grown plants

Universally applicable expression markers should indicate N-deficiency in various cultivars and growth conditions. We therefore tested the response of all 46 potential marker genes in two early senescing winter cultivars, NPZ-2 and NPZ-5, and two late senescing winter cultivars, NPZ-1005 and MSL101B, that were hydroponically grown in a greenhouse under normal N fertilization (1000 mg N), mild N-deficiency (500 mg N) and severe N-deficiency (250 mg N). The oldest leaf of each plant was harvested 27 days after transplanting (DAT) for transcription analysis. Twenty of the 46 potential marker genes showed in all cultivars qualitatively identical expression changes under both mild and severe N-deficiency compared to normal N supply. Fifteen markers were upregulated and five were downregulated (Figure 1).

To determine whether the plant response to mild and severe N-deficiency conditions is also detectable by chlorophyll measurement, SPAD values of the same leaves used for marker expression analyses were recorded from 15 DAT to 31 DAT (Figure 2). From 15 DAT to 24 DAT the SPAD values of control and N-deficient plants do not differ. At 26 DAT the SPAD value under severe N-deficiency is lower in genotypes NPZ-2 and MSL101B, but not altered in NPZ-5 and NPZ-1005, and under mild N-deficiency it is not altered in any genotype. At 28 DAT severe, but not mild N-deficiency results in a lower SPAD value in MSL101B and by trend also in NPZ-1005, whereas in NPZ-2 and NPZ-5 the SPAD values are unstable and not interpretable. At 31 DAT in MSL101B and NPZ-1005 the SPAD values are reduced under both mild and severe N-deficiency, but unstable and not interpretable in NPZ-2 and NPZ-5. These results indicate that in rapeseed expression markers can indicate N-deficiency earlier than SPAD values.

### Validation of candidate N-deficiency marker genes in field grown plants

To test the robustness of the 20 early N-deficiency marker candidates, we tested their performance on older plants in winter cultivar, Major, during the flowering/seed set phase (BBCH ~65), which were grown under very low N (20 kg N ha⁻¹) or normal N supply (120 kg N ha⁻¹), respectively. The eight oldest leaves of plants fertilized with the lower N dose all had a lower fresh weight and slightly reduced SPAD values compared to normally fertilized plants (Figure 3). Although the field-grown plants were exposed to N-deficiency for a long period and therefore it was not possible to confirm the early response of the marker genes; it was observed that 13 out of 20 potential markers were responsive to N-deficiency in a similar regulation trend, up or downregulation, that was observed in plants grown under controlled condition. This data provides a proof of principle for application of these markers in the field. However, more investigations are required to optimize the requisites of such an application.

Although the cultivar genotype, plant age, growth conditions and fertilization regime differed strongly, 13 markers showed a similar expression response to N-deficiency like in the prior tests. Figure 4 shows a compilation of the transcriptional response to N-deficiency of the 13 markers in the different genotypes and cultivation conditions.

We compared the N-deficiency response of these *B. napus* markers with that of the homologous *Arabidopsis thaliana* genes in 9-day-old seedlings grown in liquid culture (Czechowski et al. 2004). Eleven of the 13 Arabidopsis genes showed qualitatively the same transcriptional response to N-depletion like the homologous markers in rapeseed (Figure 5). This suggests that the regulation of these genes in response to N-deficiency is conserved in rapeseed and Arabidopsis.

### Example 2

### Growth conditions

The rapeseed cultivars (cv.) used are shown in Table 1. All cultivars were provided by Norddeutsche Pflanzenzucht Hans-Georg Lembke KG (NPZ), Holtsee, Germany.

**Table 1: List of the rapeseed cultivars. All the cultivars used in the current study, their description as well as relevant publications are provided.**

| **Designation** | **Description** | **Reference** |
|---|---|---|
| Apex | commercial winter cv.; N-efficient; late-senescing (stay green) | (Koeslin-Findeklee et al. 2015) |
| Capitol | commercial winter cv.; N-inefficient; early-senescing | (Koeslin-Findeklee et al. 2015) |
| Major | commercial winter cv. | (Diers et al. 1994) |
| Mozart | double-haploid spring cv. | (Franzaring et al. 2011) |
| MSL101B | male sterile Lembke line; latesenescinq | (Paulmann, 1996) |
| NPZ-2 | winter cv.; N-inefficient; early-senescing | (Koeslin-Findeklee et al. 2015) |
| NPZ-5 | hybrid cv.; N-efficient; early-senescing | (Koeslin-Findeklee et al. 2015) |
| NPZ-1005 Mercedes | winter cv.; N-efficient; late-senescing | www.bundessortenamt.de/bsa/media/File s/BlfS/BlfS_2011_11.pdf |

### Climate chamber grown plants

The cultivation of cv. Mozart is described in Franzaring et al. (2011). In brief, plants were grown in pots in climate chambers simulating natural day length, temperature and humidity profiles during spring in Southwestern Germany. Nitrogen was supplied at optimal levels equivalent to fertilization with 150 kg N ha⁻¹ ("NO") or at lower levels causing mild N-deficiency ("NL": 75 kg N ha⁻¹). The leaf harvesting workflow is reported in Safavi-Rizi et al. (2018). In brief, leaf discs from representative areas of leaves #4 and #8 were collected at 78, 85, 92, 99 and 106 days after sowing (DAS) from NO and NL plants. For each sample three biological replicates from different plants were collected, except for leaf #4 at 78 and 99 DAS with two replicates.

### Greenhouse grown plants

Cultivation of plants in hydroponics was done as described by Koeslin-Findeklee et al. (2015). Seven days after germination, seedlings of the four winter cultivars NPZ-2, NPZ-5, NPZ-1005 and MSL101B were transferred to 6-liter plastic boxes and cultivated hydroponically in a greenhouse. After vernalization and before transplanting from soil to hydroponics, initial fertilization (Flory 10^{∗}) was added to the plants (Table 2). To provide enough nutrients during plant development, macro- and micronutrients were added to the plants (Table 3). Timetable of N fertilization and other nutrients is provided in table 4. The oldest lower leaf at 27 days after transplanting (DAT) was used for the analysis. For each sample four biological replicates were used.

**Table 2. Initial fertilization including basic substrates (before transplanting). ^{∗}Flory 10: Commercially available fertilizer containing micronutrients.**

| **Nutrient** | **Form** | **Amount (mg)** |
|---|---|---|
| N | Ca(NO3)2·4H2O | 250 |
| K | K2SO4 | 100 |
| Mg | MgSO4·7H2O | 50 |
| Micronutrients | Flory 10 | 100 |
| P | KH2PO4 | 50 |

**Table 3. Amount of the nutrients added to the plants during development (after transplanting). ^{∗}Ferty 10: Commercially available fertilizer containing micronutrient.**

| **Nutrient** | **Concentration** |
|---|---|
| | In the final solution [mg/100 ml] |
| P | 2 |
| K | 20 |
| Mg | 2 |
| ^{*}Ferty 10 | 10 |

**Table 4. Timetable of N fertilization and other nutrients applied during plant development (after transplanting). X represents fertilization (for N fertilization each time 100 ml N solution containing 250 mg N was given), DAT: days after transplanting.**

| **Date (DAT)** | | **N fertilization** | |
|---|---|---|---|
| 0 | X | X | X |
| 7 | | X | X |
| 14 | | | X |
| 21 | | | X |
| Total N | 250 mg | 500 mg | 1000 mg |

| **Date (DAT)** | **other nutrients than N (see table 3)** | | |
|---|---|---|---|
| 7 | X | | |
| 11 | X | | |
| 18 | X | | |

### Field grown plants

Rapeseed winter cv. Major were vernalized 8-12 weeks at 4 °C under short day conditions (8 h day, 16 h night) and then transplanted to the open field. All plants were fertilized with 20 kg N/ha in autumn. The normal N-supplied plants obtained two additional fertilizer doses of 40 kg N/ha in early spring before the emergence of side shoots (BBCH 30) and another 60 kg N/ha at the emergence of the single closed flowers on the main inflorescence (BBCH 55). NH4NO3 fertilizer was applied in the granulate form (Yara, Dülmen, Germany). At BBCH ~65, SPAD values were recorded for the eight oldest leaves on three plants and for marker expression analysis samples were harvested from the fourth oldest leaf of three plants.

### Example 3

### Brassica napus microarray design and annotation

Microarray design, annotation and processing of the data was reported by Safavi-Rizi et al. (2018) and Koeslin-Findeklee et al. (2015). In brief, an Agilent 8×60k microarray was designed for 59,577 putative rapeseed genes assembled from contigs built with publicly available ESTs (NCBI Gene Expression Omnibus repository GPL19044, expression data series entry GSE97653). By BLASTing against the *Arabidopsis thaliana* cDNA collection (TAIR10) the closest Arabidopsis homologs were identified (Safavi-Rizi et al. 2018).

### RT-PCR primer design for B. napus genes

Specific RT-PCR primers were designed for the putative *B. napus* genes using QuantPrime (Arvidsson et al. 2008).

### RNA extraction, cDNA synthesis and qPCR assay

Total RNA was isolated from *B. napus* leaves using a hot phenol method (Shirzadegan et al. 1991). RNA concentration was quantified using a spectrophotometer (NanoDrop ND-1000, Thermo Scientific, Wilmington, DE, USA). 1 µg DNaseI-digested total RNA was used for cDNA synthesis using oligo-(dT)18 and the RevertAid H Minus First Strand kit (Thermo Scientific, Waltham, USA). cDNA was synthesized using Superscript III (Life Technologies) following manufacturer's instructions. RT-PCR reactions were performed in 5 µl total volume including 2.5 µl SYBR Green master mix (Thermo Fisher Scientific), 0.5 µM forward and reverse primers and 0.5µl cDNA. *UP1* and *UBC9* were used as reference genes (Chen et al. 2010). The following standard thermal profile was used for all qPCRs: 95°C for 10 min; 40 cycles of 95°C for 15 sec and 60°C for 1 min. Relative expression changes were calculated by the 2^{-ΔΔCT} method.

## Claims

1. A method for early determination of nitrogen (N) deficiency in rapeseed *(Brassica napus L.*) by using the gene expression of at least two marker genes of a set of marker genes, wherein said set comprises the *Brassica napus* expression marker genes
| |
|---|
| BnaC03g77120D |
| BnaA02g27980D |
| BnaA03g40690D |
| BnaA06g37010D |
| BnaA06g22900D |
| BnaA03g40160D |
| BnaC07g37670D |
| BnaC05g14940D |
| BnaA09g04420D |
| BnaC01g35070D |
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |
wherein at least one of the two marker genes BnaA01g24440D (ELIP1) or BnaC01g03140D (DRL1) has to be used, and wherein the method comprises:
• Determining the level of expression of said at least two marker genes in a biological sample taken from said rapeseed,
• Comparing the level of expression of said at least two marker genes in a biological sample taken from said rapeseed with the level of expression of said genes in a second biological sample taken from a rapeseed plant that was grown under optimal N supply of preferably 120 kg N ha⁻¹ t to 150 kg N ha⁻¹ and/or with a predetermined threshold level of expression of said at least two marker genes,
wherein a deviation of the level of expression as determined by said comparison, which is an upregulation of BnaA09g04420D and/or BnaA02g32390D and/or BnaA07g29070D and/or BnaA02g02940D and/or BnaA03g48610D and/or a downregulation of BnaC03g77120D and/or BnaA02g27980D and/or BnaA03g40690D and/or BnaA06g37010D and/or BnaA06g22900D and/or BnaA03g40160D and/or BnaC07g37670D and/or BnaC05g14940D and/or BnaC01g35070D and/or BnaC01g14360D and/or BnaA09g28910D and/or BnaC07g41470D and/or BnaC06g30680D and/or BnaA01g24440D and/or BnaC01g03140D, is indicative for a nitrogen deficiency in said rapeseed,
and wherein the term early means before the leaves of the plant show phenotypical changes, in particular discoloration of the leaves due to nitrogen deficiency.

2. The method for early determination of N deficiency in rapeseed *(Brassica napus L.*) by using the gene expression of at least two marker genes of a set of marker genes according to claim 1, wherein the expression profile is an RNA profile.

3. The method for early determination of nitrogen deficiency in rapeseed *(Brassica napus L.*) by using the gene expression of at least two marker genes of a set of marker genes according to claims 1 to 2, wherein an increase of the level of expression of said gene in the biological sample compared to the level of expression of said gene in the second biological sample taken from a rapeseed plant that was grown under optimal nitrogen supply of preferably 120 kg N ha⁻¹ t to 150 kg N ha⁻¹ and/or with a predetermined threshold means the biomarker gene expression is up-regulated,
wherein a decrease of the gene expression level of said gene in the biological sample compared to the level of expression of said gene in the second biological sample taken from a rapeseed plant that was grown under optimal nitrogen supply of preferably 120 kg N ha⁻¹ t to 150 kg N ha⁻¹ and/or with a predetermined threshold means the biomarker gene expression is downregulated,
and wherein an up-regulation or down-regulation of the gene expression of said gene is indicative for said nitrogen deficiency in rapeseed.

4. A method for monitoring nitrogen deficiency in rapeseed *(Brassica napus L.*) by performing a method for early determination of nitrogen (N) deficiency in rapeseed according to claims 1-3 at least twice and wherein said method is used to monitor the response of a plant being treated for nitrogen deficiency by supplying nitrogen.

5. A method for determining the need of plant nitrogen fertilization in rapeseed *(Brassica napus L.*) by performing a method for early determination of nitrogen (N) deficiency in rapeseed according to any of claims 1-4, and thereby selecting a rapeseed that displays nitrogen deficiency and preferably optimizing the application with nitrogen for the selected plant by increasing the nitrogen amount.

6. An assay for early determination of nitrogen (N) deficiency in rapeseed *(Brassica napus L.*) comprising *Brassica napus* expression marker genes:
| |
|---|
| BnaC03g77120D |
| BnaA02g27980D |
| BnaA03g40690D |
| BnaA06g37010D |
| BnaA06g22900D |
| BnaA03g40160D |
| BnaC07g37670D |
| BnaC05g14940D |
| BnaA09g04420D |
| BnaC01g35070D |
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |
,
wherein the assay preferably also comprises an assay for RNA extraction and a microarray or a PCR based method.

7. Use of gene expression of at least two marker genes of a set of marker genes for detecting and/or monitoring early nitrogen (N) deficiency in rapeseed *(Brassica napus L.),*
wherein the gene expression of said marker genes is nitrogen responsive and is used as an early indicator for the nitrogen status in the plant, wherein said set comprises *Brassica napus* expression marker genes
| |
|---|
| BnaC03g77120D |
| BnaA02g27980D |
| BnaA03g40690D |
| BnaA06g37010D |
| BnaA06g22900D |
| BnaA03g40160D |
| BnaC07g37670D |
| BnaC05g14940D |
| BnaA09g04420D |
| BnaC01g35070D |
| BnaA02g32390D |
| BnaA07g29070D |
| BnaC01g14360D |
| BnaA09g28910D |
| BnaC07g41470D |
| BnaC06g30680D |
| BnaA02g02940D |
| BnaA03g48610D |
| BnaA01g24440D |
| BnaC01g03140D |
wherein at least one of the marker genes BnaA01g24440D (ELIP1) or BnaC01g03140D (DRL1) from *Brassica napus L.* has to be detected.
